(19) Europäisches Patentamt
European Patent Office
Office européen des brevets

(11) Numéro de publication: **0 268 507 B1**

# (12) FASCICULE DE BREVET EUROPEEN

(45) Date de publication de fascicule du brevet: **06.03.91**

(51) Int. Cl.[5]: **C07C 327/20**, C07C 327/22

(21) Numéro de dépôt: **87402267.6**

(22) Date de dépôt: **12.10.87**

## (54) Procédé de fabrication des thiochloroformiates d'alkyle.

(30) Priorité: **17.10.86 FR 8614407**

(43) Date de publication de la demande: **25.05.88 Bulletin 88/21**

(45) Mention de la délivrance du brevet: **06.03.91 Bulletin 91/10**

(84) Etats contractants désignés: **AT BE DE ES GB NL**

(56) Documents cités:
**EP-A- 0 053 981**
**FR-A- 2 462 423**
**US-A- 4 340 746**

(73) Titulaire: **SOCIETE NATIONALE DES POUDRES ET EXPLOSIFS**
**12, quai Henri IV**
**F-75181 Paris Cédex 04(FR)**

(72) Inventeur: **Gauthier, Patricia**
**Chemin des Acacias**
**Cerny F-91590 La Ferte Alais(FR)**
Inventeur: **Malfroot, Thierry**
**17, Chemin des Jardins**
**F-91100 Saintry Sur Seine(FR)**
Inventeur: **Senet, Jean-Pierre**
**79, rue de la Gare Herbeauvilliers-Buthiers**
**F-77760 La Chapelle La Reine(FR)**

(74) Mandataire: **Jomard, Annick et al**
**SNPE - Service Propriété Industrielle 12, Quai Henri IV**
**F-75181 Paris Cedex 04(FR)**

**EP 0 268 507 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet un nouveau procédé de préparation des thiochloroformiates.

Il est depuis longtemps connu de préparer les thiochloroformiates par réaction du phosgène sur un mercaptan, en présence d'un catalyseur.

Le schéma réactionnel est le suivant :

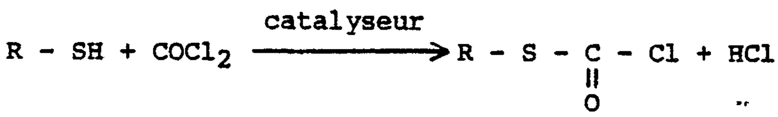


La présence d'un catalyseur est indispensable pour obtenir le thiochloroformiate dans des conditions économiquement acceptables. Le charbon actif a été largement utilisé, mais il présente plusieurs inconvénients. Son recyclage est difficile car il perd son pouvoir catalytique. De plus, de fines particules restent en suspension dans le produit final, rendant nécessaire une purification délicate en fin de synthèse.

Plusieurs types de catalyseurs organiques ont aussi été proposés :

- les amides (brevets US 3 277 143 et US 4 340 746)
- les amines secondaires et leurs chlorhydrates (brevets US 4 273 725 et FR 2 462 423)
- les amines tertiaires (brevet US 3 277 143)
- les sels d'ammonium quaternaires (brevet US 4 268 456 et FR 2 462 423)
- certaines résines échangeuses d'anions (brevet FR 2 462 423)
- les urées et leurs produits de réaction avec le phosgène ou un thiochloroformiate (brevet US 4 340 746).

Tous ces catalyseurs présentent, à des degrés divers, les inconvénients suivants :

- Pour obtenir une cinétique suffisante, il faut au moins 0,01 équivalent molaire de catalyseur. Ceci implique une purification par distillation en fin de réaction. Or cette distillation est très difficile à effectuer dans le cas des thiochloroformiates lourds.
- Les rendements obtenus sont variables et parfois faibles.
- L'emploi de la plupart de ces catalyseurs entraîne la formation de sous-produits gênants (chlorures de carbamyle, goudrons, boues...), rendant délicate et fastidieuse la purification des produits finis.

Il existe donc un besoin non satisfait de disposer d'un catalyseur efficace et ne présentant pas les inconvénients exposés.

La présente invention a pour objet un procédé de fabrication des thiochloroformiates, utilisant de tels catalyseurs.

Le procédé selon l'invention est un procédé de fabrication de thiochloroformiates d'alkyle par action du phosgène sur un mercaptan, en présence d'un catalyseur, caractérisé en ce que ledit catalyseur est un chlorure d'hexaalkylguanidinium ou bien le chlo rhydrate correspondant, de formule générale :

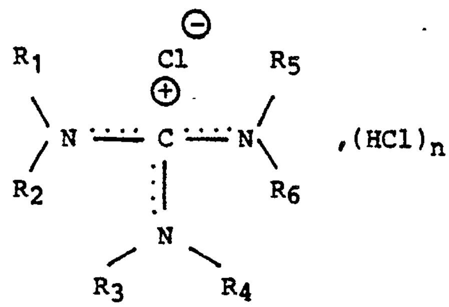

dans laquelle $R_1$, $R_2$, $R_3$, $R_4$, $R_5$, $R_6$ sont identiques ou différents et représentent des radicaux alkyle de $C_1$ à $C_4$ et dans laquelle n peut prendre les valeurs 0 ou 1.

Un avantage de ces catalyseurs est que la quantité nécessaire est beaucoup plus faible que celles décrites dans les autres procédés. Dans le procédé selon l'invention, cette quantité est comprise entre $10^{-3}$ et $10^{-2}$ équivalent molaire de catalyseur par rapport au thiol.

Les thiochloroformiates ainsi obtenus ne contiennent donc pratiquement pas de catalyseur en tant qu'impuretés.

Cet avantage permet, dans de nombreux cas, d'éviter une distillation particulièrement difficile, voir

impossible sans dégradation. Cet avantage s'applique surtout aux thiochloroformiates dont le radical alkyle comporte entre 6 et 20 atomes de carbone et préférentiellement entre 6 et 12 atomes de carbone.

Selon une variante particulièrement avantageuse de l'invention, $R_1$, $R_2$, $R_3$, $R_4$, $R_5$, $R_6$ sont identiques et représentent le radical méthyle.

On décrit ci-après l'invention de manière détaillée.

Le procédé selon l'invention est donc un procédé de synthèse de thiochloroformiates par réaction du phosgène sur un mercaptan ou thiol, en présence d'un catalyseur, selon le schéma réactionnel :

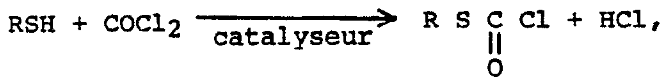


dans lequel
R représente un reste alkyle ou cycloalkyle.

Le procédé selon l'invention s'applique aux thiols RSH dont le radical R est un reste alkyle saturé, linéaire ou ramifié, ou cycloalkyle, comportant de 1 à 20 atomes de carbone.

Le catalyseur utilisé est un chlorure d'hexaalkylguanidinium ou bien le chlorhydrate correspondant de formule générale (I)

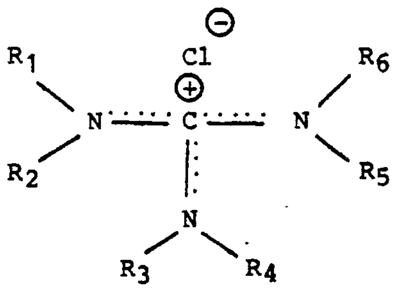 , $(HCl)_n$ (I)

dans laquelle $R_1$, $R_2$, $R_3$, $R_4$, $R_5$ et $R_6$, identiques ou différents, représentent des radicaux alkyles comportant de 1 à 4 atomes de carbone et dans laquelle n peut prendre soit la valeur 0 soit la valeur 1.

Les catalyseurs préférés dans le cadre de la présente invention sont ceux pour lesquels les six radicaux $R_1$, $R_2$, $R_3$, $R_4$, $R_5$ et $R_6$ sont identiques et représentent le groupe méthyle, à savoir le chlorure d'hexaméthylguanidinium ou son chlorhydrate de formule générale (II) :

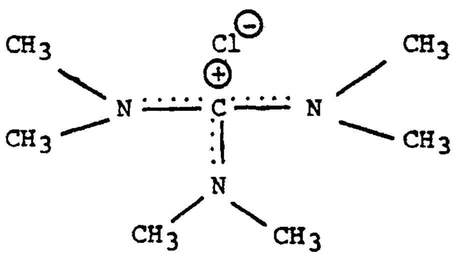 ,$(HCl)_n$ (II)

avec n = O ou 1.

En effet les composés de formule (II) utilisés comme catalyseurs de phosgénation des mercaptans RSH dont le radical R comporte un nombre d'atomes de carbone compris entre 6 et 20 précipitent en fin de réaction après élimination du phosgène par dégazage. Il est donc possible dans ce cas d'obtenir des thiochloroformiates parfaitement purs, exempts de toute trace de catalyseur, par simple filtration.

Les catalyseurs selon l'invention, qui sont par ailleurs décrits comme catalyseurs de phosgénation des acides en chlorures d'acide dans la demande de brevet européen EP-A-213976 au nom de la demanderesse, peuvent se préparer de façon connue selon plusieurs procédés. On peut, par exemple, les préparer à partir d'une urée ou thiourée qui est phosgénée pour obtenir le chlorure de chloroformamidinium (Chem. Ber. 97, p 1232, 1964) que l'on met ensuite à réagir avec une amine (Synthesis 1983, 11, pages 904-905) ; ou bien par réaction d'une thiourée avec un chlorure de carbamyle (Liebigs Ann. Chem., 1984, pages 108-126) ; ou encore à partir de pentaalkylguanidines que l'on fait réagir sur un dérivé halogéné (Houben Weyl,

VIII, pages 100 et 186, 1952), les pentaguanidines étant elles-mêmes obtenues par réaction d'un isocyanatodihalogénure sur une amine secondaire (brevet FR 1 453 438).

Les chlorhydrates peuvent être préparés soit par addition d'acide chlorhydrique sur le chlorure d'hexaalkylguanidinium, soit, lorsque l'on utilise la voie faisant appel à un chlorure de chlorofor mamidinium, par réaction de ce chlorure de formamidinium sur une amine secondaire suivie, sans séparation des produits obtenus, par une phosgénation.

La réaction de phosgénation des mercaptans ou thiols selon l'invention peut se faire en l'absence de solvant, ou en présence d'un solvant inerte vis-à-vis du phosgène et apte à dissoudre à la fois le mercaptan et le phosgène. Ce solvant peut être un solvant aliphatique chloré tel que dichlorométhane, ou bien un solvant aromatique tel que le chlorobenzène, le toluène ou un xylène. Le toluène convient bien.

Néanmoins, la solution préférée est la phosgénation du mercaptan sans solvant.

Selon un autre avantage de l'invention la réaction peut être conduite à température modérée, comprise entre 30 °C et 50 °C, en raison de la grande efficacité des catalyseurs utilisés.

D'une manière générale, on travaille avec un excès de phosgène, pour éviter la formation de produits parasites tels que des carbonates par exemple. De préférence la réaction selon l'invention se fait en présence d'un excès de phosgène de 5 à 10 % en moles.

Grâce au procédé selon l'invention, on obtient des thiochloroformiates d'alkyle pratiquement exempts d'impuretés, avec des conditions opératoires adoucies et des opérations de purification simplifiées .

De plus le catalyseur peut être facilement recyclé notamment lorsqu'il est récupéré par filtration.

Les thiochloroformiates sont des composés très connus dont les applications sont nombreuses, par exemple comme intermédiaires pour les industries phytosanitaires. Le procédé selon l'invention permet d'accéder aux thiochloroformiates conduisant aux thiocar bamates rentrant dans un grand nombre de compositions herbicides et pesticides. Selon un mode de réalisation particulièrement avantageux du procédé, on peut obtenir des thiochloroformiates d'alkyles lourds, jusqu'alors très difficiles voir impossibles à obtenir par les procédés connus.

Le procédé selon l'invention est particulièrement bien adapté à la préparation du thiochloroformiate de n-octyle, de formule (3)

$$CH_3(CH_2)_7-S-\underset{\underset{O}{\|}}{C}-Cl$$

qui est un intermédiaire important dans l'industrie des pesticides.

## Exemple

Préparation du chloroformiate de n-octane thiol.

Cet exemple est relatif à la synthèse du n-octylthiochloroformiate par réaction du n-octane thiol sur le phosgène en présence du chlorhydrate de chlorure d'hexaméthyl guanidinium comme catalyseur.

### A/ Préparation du catalyseur

Le catalyseur est préparé par phosgénation de la tétraméthylurée de manière à obtenir le chlorure de tétraméthyl chloroformamidinium que l'on fait ensuite réagir avec la diméthylamine pour finir par une phosgénation du mélange réactionnel selon le schéma suivant :

a) 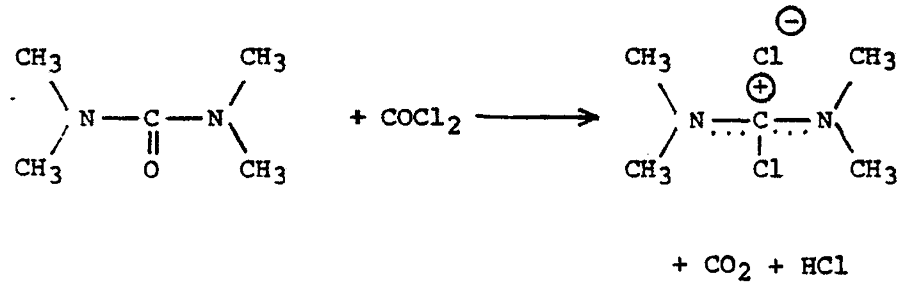

b) 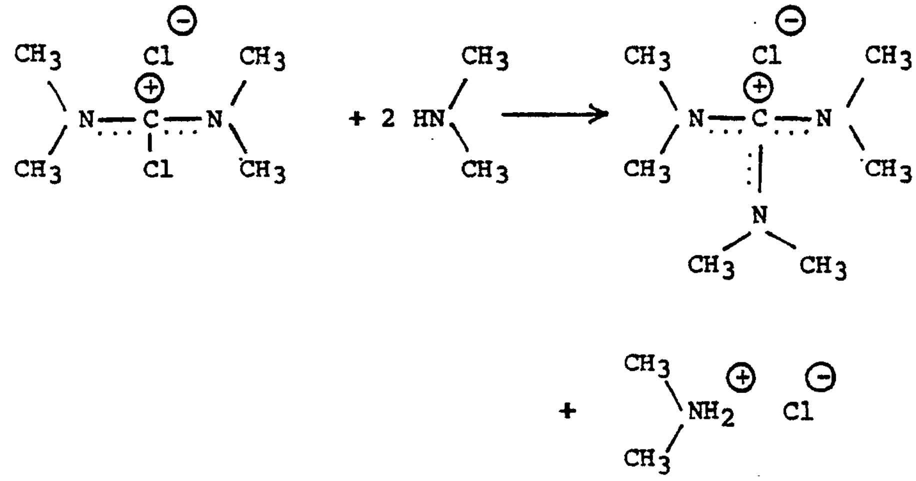

c) 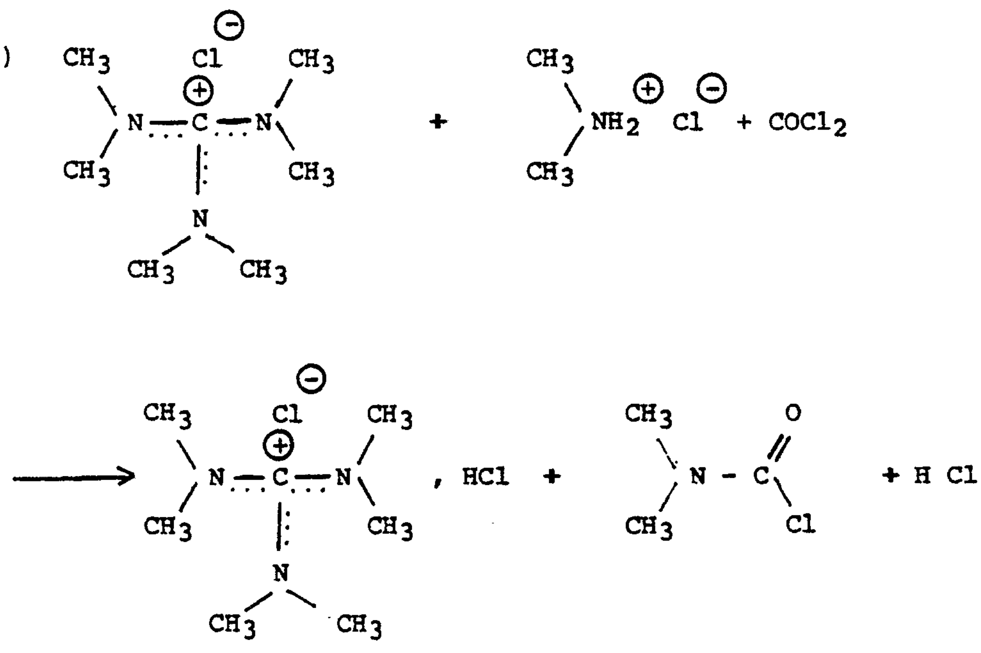

0,5 mole de tétraméthylurée est phosgénée dans 130 cm³ de di-chloro-1,2 éthane à 80° C en trois heures en présence d'un excès molaire de phosgène de 25 %. Après addition du phosgène l'agitation est maintenue à 80° C pendant quatre heures. Le phosgène en excès est ensuite éliminé du milieu réactionnel par un dégazage sous pression réduite.

Dans un réacteur de 500 ml équipé d'une agitation mécanique, d'un thermomètre, d'un réfrigérant utilisant le mélange carboglace-chlorure de méthylène et d'un tube d'introduction, on ajoute en deux heures et demie 1,1 moles de diméthylamine gazeuse au milieu réactionnel précédent. La réaction est très exothermique et la température passe de 20° C à 82° C.

L'ensemble est laissé sous agitation pendant une heure. Le milieu est hétérogène mais facile à agiter. On peut vérifier par infra-rouge la disparition caractéristique de la bande C ⊕ -N à 1650 $cm^{-1}$ du chloroformamidinium et l'apparition à 1580 $cm^{-1}$ et 1600 $cm^{-1}$ des bandes du sel de guanidinium.

Le milieu réactionnel est alors porté à reflux entre 90° C et 95° C et on ajoute 0,75 mole de phosgène gazeux en deux heures et demie. L'agitation est ensuite maintenue à la température de 90° C pendant trois heures et demie.

L'analyse infra-rouge montre la présence de chlorure de carbamyle par une bande de 1740 $cm^{-1}$. Ce composé est dosé par chromatographie CPG avec étalon interne. On peut ainsi déterminer la fin de réaction.

Le phosgène en excès est ensuite éliminé par dégazage sous pression réduite. Après élimination du dichloro-1,2 éthane sous 12 mm de mercure à une température comprise entre 50-60° C, le résidu obtenu est repris avec 100 $cm^3$ d'hexane. Le chlorhydrate du chlorure d'hexaméthyl guanidinium précipite.

Après filtration et lavages avec deux fois 200 $cm^3$ d'hexane, le produit est sèché sous vide de 1 mm de mercure à température ambiante jusqu'à poids constant.

Le produit présente les caractéristiques suivantes :

masse obtenue : 104 g
masse théorique : 108 g
rendement : 96 %
point de fusion : 105° C au banc Kofler
taux de chlore hydrolysable obtenu : 31 %
taux de chlore hydrolysable théorique : 32,87 %

B/ Préparation du chloroformiate de n-octane thiol

Dans un réacteur de 500 ml surmonté d'un réfrigérant à - 70° C, on in troduit 146 g (1 mole) d'octane thiol et 0,324 g (0,0015 mole) de chlorhydrate de chlorure d'hexaméthyl guanidinium. Le phosgène (1,5 moles) est introduit gazeux en deux heures dans le mélange réactionnel maintenu à 37° C (± 2° C). L'addition terminée, l'agitation est poursuivie 3 heures à cette température. La réaction est alors terminée.

Après dégazage soigneux à 30° C sous 30 mm de mercure, le catalyseur qui précipite est séparé du thiochloroformiate par filtration, la température du milieu réactionnel étant abaissée à 8-10° C, sur filtre de type SARTORIUS ® avec une toile téflon à mailles de 5 micromètres.

Après lavage du catalyseur à l'hexane et sèchage sous pression réduite (1 mm de mercure) à température ambiante on récupère 0,3049 g de catalyseur soit en masse 94 % de la quantité engagée.

Le thiochloroformiate de n-octyle obtenu avec un rendement de 100% après séparation du catalyseur présente les caractéristiques suivantes :

- taux de chlore :
  théorique : 17,026%
  trouvé : 17,03 - 17,08 % (pureté 100 %)
- chromatographie en phase gazeuse : pas d'impureté
- spectrométrie infra-rouge : bande C = 0 à 1770 $cm^{-1}$
- coloration : 20 APHA
- absence de produits en suspension après 2 h à - 30° C et 10 jours à température ambiante.

Un essai de recyclage du catalyseur récupéré a été effectué. Employé à raison de 0,13 % molaire, il a permis la phosgénation d'un nouveau lot d'octaine thiol dans les conditions précédentes en 7 h 15 (taux de transformation : 99 %).

## Revendications

1. Procédé de fabrication de thiochloroformiates par action du phosgène sur un mercaptan, en présence d'un catalyseur, caractérisé en ce que ledit catalyseur est un chlorure d'hexaalkylguanidinium ou bien le chlorhydrate correspondant de formule générale :

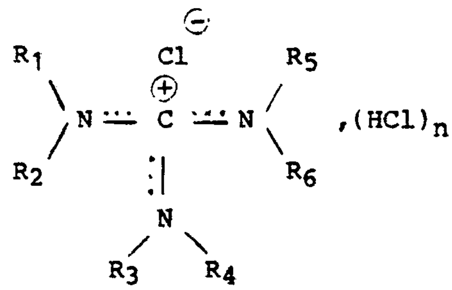

dans laquelle $R_1$, $R_2$, $R_3$, $R_4$, $R_5$, $R_6$ sont identiques ou différents et représentent des radicaux alkyle de $C_1$ à $C_4$ et dans laquelle n peut prendre les valeurs 0 ou 1.

2. Procédé selon la revendication 1, caractérisé en ce que $R_1$, $R_2$, $R_3$, $R_4$, $R_5$, $R_6$ sont identiques et représentent le méthyle.

3. Procédé selon l'une quelconque des revendications précédentes, caractérisé en ce que la quantité de catalyseur utilisé est comprise entre $10^{-3}$ et $10^{-2}$ équivalent par rapport au thiol.

4. Procédé selon l'une quelconque des revendications précédentes, caractérisé en ce que la température de la réaction est comprise entre 30 et 50° C.

5. Procédé selon l'une quelconque des revendications précédentes, caractérisé en ce que le mercaptan est de formule générale R-SH dans laquelle R est un radical alkyle ou cycloalkyle de $C_6$ à $C_{20}$.

6. Procédé selon la revendication 5 caractérisé en ce qu'on utilise comme catalyseur le chlorure d'hexaméthylguanidinium ou son chlorhydrate et qu'on le sépare du thiochloroformiate en fin de réaction par filtration.

7. Procédé selon l'une quelconque des revendications précédentes caractérisé en ce que la réaction se fait avec un excès de phosgène de 5 à 10 % en moles.

## Claims

1. Process for the preparation of thiochloroformates by reaction of phosgene with a mercaptan in the presence of a catalyst, characterised in that the said catalyst is a hexaalkylguanidinium chloride or the corresponding chlorohydrate of the general formula:

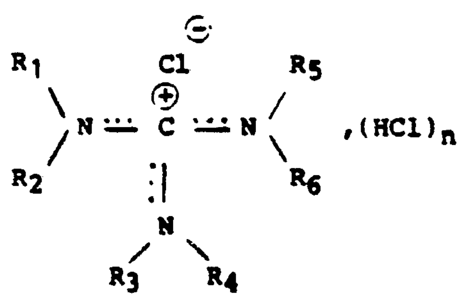

in which $R_1$, $R_2$, $R_3$, $R_4$, $R_5$ and $R_6$ are identical or different and represent $C_1$ to $C_4$-alkyl radicals and in which n can adopt the values 0 or 1.

2. Process according to Claim 1, characterised in that $R_1$, $R_2$, $R_3$, $R_4$, $R_5$ and $R_6$ are identical and represent methyl.

3. Process according to any one of the preceding claims, characterised in that the amount of catalyst used is between $10^{-3}$ and $10^{-2}$ equivalent, relative to the thiol.

4. Process according to any one of the preceding claims, characterised in that the reaction temperature is between 30 and 50 °C.

5. Process according to any one of the preceding claims, characterised in that the mercaptan has the general formula R-SH, in which R is a $C_6$ to $C_{20}$-alkyl or cycloalkyl radical.

6. Process according to Claim 5, characterised in that the catalyst used is hexamethylguanidinium chloride or its chlorohydrate and that at the end of the reaction it is separated from the thiochloroformate by filtration.

7. Process according to any one of the preceding claims, characterised in that the reaction is carried out with a phosgene excess of 5 to 10 mol %.

## Ansprüche

1. Verfahren zur Herstellung von Chlorameisensäurethioestern durch Umsetzung von Phosgen mit einem Thiol in Gegenwart eines Katalysators,
**dadurch gekennzeichnet,** daß
als Katalysator ein Hexaalkylguanidiniumchlorid oder das entsprechende Hydrochlorid der allgemeinen Formel

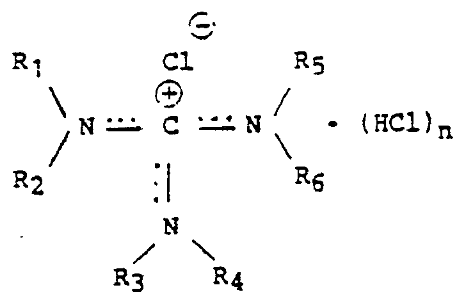

verwendet wird,
in der $R_1$, $R_2$, $R_3$, $R_4$, $R_5$, $R_6$ gleich oder verschieden sind und $C_{1-4}$-Alkylgruppen darstellen
und
n die Werte 0 oder 1 bedeutet.

2. Verfahren nach Anspruch 1, dadurch gekennzeichnet, daß $R_1$, $R_2$, $R_3$, $R_4$, $R_5$, $R_6$ gleich sind und Methyl darstellen.

3. Verfahren nach einem der vorhergehenden Ansprüche, dadurch gekennzeichnet, daß die Menge des verwendeten Katalysators $10^{-3}$ bis $10^{-2}$ Äquivalent, bezogen auf Thiol, beträgt.

4. Verfahren nach einem der vorhergehenden Ansprüche, dadurch gekennzeichnet, daß die Reaktionstemperatur 30 bis 50 °C beträgt.

5. Verfahren nach einem der vorhergehenden Ansprüche, dadurch gekennzeichnet, daß das Thiol die allgemeine Formel R-SH besitzt, in der R $C_{6-20}$-Alkyl- oder $C_{6-20}$-Cycloalkylrest bedeutet.

6. Verfahren nach Anspruch 5, dadurch gekennzeichnet, daß als Katalysator Hexamethylguanidiniumchlorid oder sein Hydrochlorid verwendet und der Katalysator am Ende der Reaktion durch Filtration vom Chlorameisensäurethioester abgetrennt wird.

7. Verfahren nach einem der vorhergehenden Ansprüche, dadurch gekennzeichnet, daß die Reaktion bei einem Phosgenüberschuß von 5 bis 10 Mol-% durchgeführt wird.